# EUROPEAN PATENT APPLICATION

(11) **EP 4 628 091 A1**
(43) Date of publication of application: **08.10.2025**
(21) Application number: 23898325.8
(22) Date of filing: 30.11.2023
(51) Int. Cl.: A61K 36/28, A61K 36/53, A61P 29/00, A61P 19/02, A23L 33/105

(54) **COMPOSITION FOR PREVENTION, TREATMENT, OR AMELIORATION OF INFLAMMATORY DISEASE**

(30) Priority: 01.12.2022 KR 20220165901
(71) Applicant: Woore Green Science Co., Ltd., Ansan-si, Gyeonggi-do 15409 (KR)
(72) Inventor: LEE, Hyun Yong, Yongin-si Gyeonggi-do 16955 (KR); KWUN, Duk Won, Siheung-si Gyeonggi-do 15068 (KR); KIM, Sung Hun, Uiwang-si Gyeonggi-do 16016 (KR); LEE, Sang Hyun, Daejeon 34985 (KR); KIM, Jong Hwa, Ansan-si Gyeonggi-do 15589 (KR); KANG, Su Ji, Yongin-si Gyeonggi-do 16817 (KR); KIM, Kyeong Jin, Siheung-si Gyeonggi-do 15118 (KR); JEONG, Yu Mi, Ansan-si Gyeonggi-do 15341 (KR); CHOI, Yoon Seon, Incheon 21654 (KR); JEON, Ki Beom, Ansan-si Gyeonggi-do 15441 (KR); KIM, Yoon Jeong, Ansan-si Gyeonggi-do 15409 (KR)
(74) Representative: Zacco Sweden AB
(86) International application number: PCT/KR2023/019504
(87) International publication number: WO 2024/117801

(57) **Abstract**

A pharmaceutical composition for preventing or treating an inflammatory disease of the present invention contains marigold and basil extracts as active ingredients. In addition, a functional food composition for preventing or improving an inflammatory disease of the present invention contains marigold and basil extracts as active ingredients.

## Description

### [Technical Field]

The present invention relates to a composition for preventing, treating, or improving an inflammatory disease. More particularly, the present invention relates to a composition using a plant material, which can effectively prevent, treat, or improve an inflammatory disease by regulating the expression of inflammatory disease-related factors.

### [Background Art]

An inflammatory response is a type of biological response to random stimuli such as tissue damage or microbial infection, and is essential for normal body maintenance. However, when such inflammatory responses occur excessively, tissue or organs in the body in the area with inflammation cannot maintain their original functions, causing pain and fever. Diseases caused by the excessive inflammatory responses are called inflammatory diseases. Although much research has been done on anti-inflammatory agents to cure these inflammatory diseases, the demand for safer and more effective anti-inflammatory agents continues.

A representative inflammatory disease is arthritis. Arthritis is a disease in which damage or inflammation occurs in joints where bones meet due to various causes. Many people suffer from arthritis, which may have an adverse effect on social activities, especially by hindering the patient's mobility. Particularly, degenerative arthritis among arthritis is a progressive and irreversible degenerative disease that reduces the quality of life, and is the most common degenerative disease that affects 8 out of 10 elderly people. Particular, degenerative arthritis is the most representative degenerative disease that causes pain, impaired daily life activities, and movement disorders, thereby lowering the quality of life and consuming astronomical social costs for treatment, etc. However, there is currently no therapeutic agent for it.

Degenerative arthritis is a disease that brings degeneration of articular cartilage tissue and structural changes of subchondral bone. Its pathological cause has not yet been clearly identified, but the main causes include the wear of cartilage due to its repeated use in the course of aging, genetics, damage to cartilage tissue from impact, pressure caused by excess weight, and weakened muscles around the knees. In particular, innate factors such as aging and genetics and mechanical factors such as obesity, injury, and muscle weakening activate biochemical pathways that lead to degenerative arthritis.

The inventors discovered effective materials for the above-described inflammatory diseases from plant materials to develop a pharmaceutical composition and a functional food composition that can effectively prevent, treat or improve arthritis, especially degenerative arthritis, and confirmed that the combination of marigold and basil is very effective on these diseases. Thus, the present invention was completed.

### [Disclosure]

### [Technical Problem]

The present invention is directed to providing a pharmaceutical composition and a functional food composition that can effectively prevent, treat, or improve an inflammatory disease, such as arthritis, especially degenerative arthritis, by using plant materials.

### [Technical Solution]

One aspect of the present invention provides a pharmaceutical composition for preventing or treating an inflammatory disease, which contains marigold and basil extracts as active ingredients.

Another aspect of the present invention provides a functional food composition for preventing or improving an inflammatory disease, which contains marigold and basil extracts as active ingredients.

### [Advantageous Effects]

A composition of the present invention uses a plant material, and can effectively prevent, treat or improve an inflammatory disease, such as arthritis, especially degenerative arthritis, based on the inhibitory effect of the increased expression of related factors such as MMP3, MMP13, IL-6, and COX-2 due to an inflammatory response. Particularly, the composition of the present invention can exhibit a synergistic effect from the combination of two types of plant materials.

### [Description of Drawings]

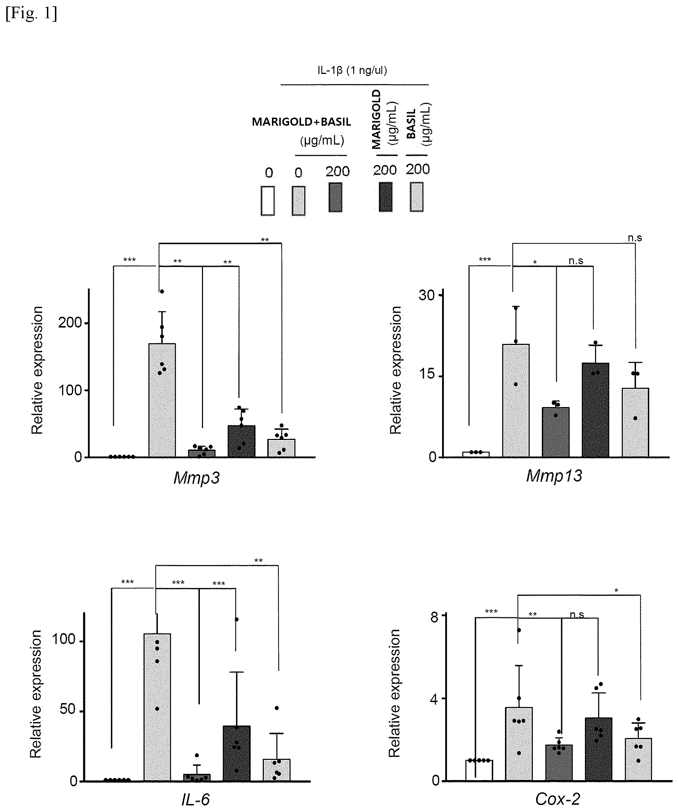

FIG. 1 shows the expression levels of MMP3, MMP13, IL-6 and COX-2, obtained by qRT-PCR, when chondrocytes isolated from mouse knee joints are treated with IL-1β (1 ng/mL) to cause degenerative arthritis, and then treated with a mixture of a marigold extract and a basil extract (marigold + basil) in a weight ratio of 1:1, with a marigold extract alone (marigold), and with a basil extract alone (basil). *P<0.05, **P<0.01, ***P<0.001, ****P<0.0001, n.s.= not significant.

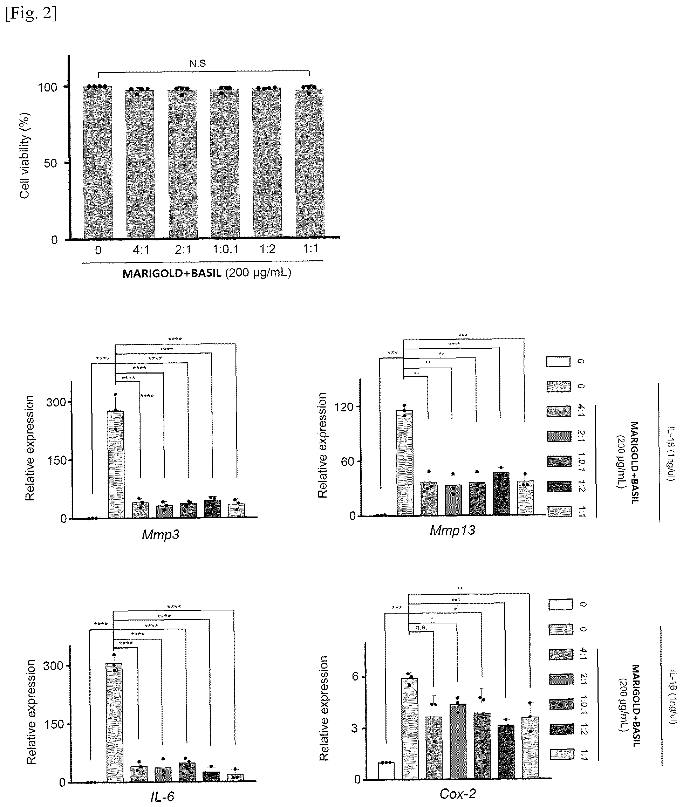

FIG. 2 shows the expression levels of MMP3, MMP13, IL-6, and COX-2, obtained by qRT-PCR, when chondrocytes isolated from mouse knee joints are treated with IL-1β (1 ng/mL) to cause degenerative arthritis, and then treated with a mixture of a marigold extract and a basil extract in a weight ratio of 4:1, 2:1, 1:0.1, or 1:2. *P<0.05, **P<0.01, ***P<0.001, ****P<0.0001, n.s.=not significant.

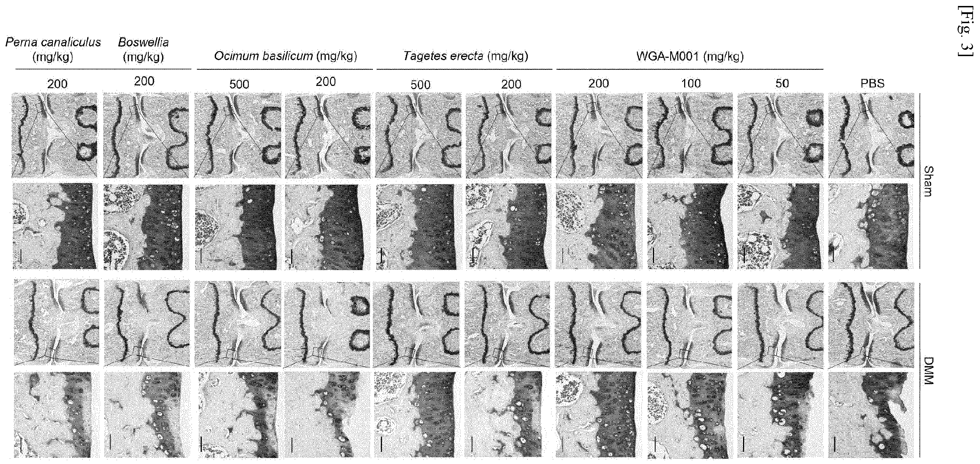

FIG. 3 shows the results of observing the therapeutic effect by concentration-dependent administration of experimental materials in mice in which osteoarthritis is induced by destabilization of medial meniscus (DMM). FIG. 3 shows the photographs of joints, cartilage, and synovium stained with Safranin O after administering an experimental material to each of normal group mice (Sham) and mice with osteoarthritis induced by DMM at different concentrations for 10 weeks and conducting an autopsy of the mice. [PBS, a negative control administered phosphate-buffered saline (PBS); WGA-M001, a group administered a complex of a marigold extract and a basil extract; *Tagetes erecta,* a group administered only marigold extract; *Ocimum basilicum,* a group administered only basil extract; *Boswellia,* a positive control administered *Boswellia* extract (currently used as a functional raw material for joint health); and *Perna canaliculus,* a positive control administered green lipped mussel extract (currently used as a joint health functional ingredient)].

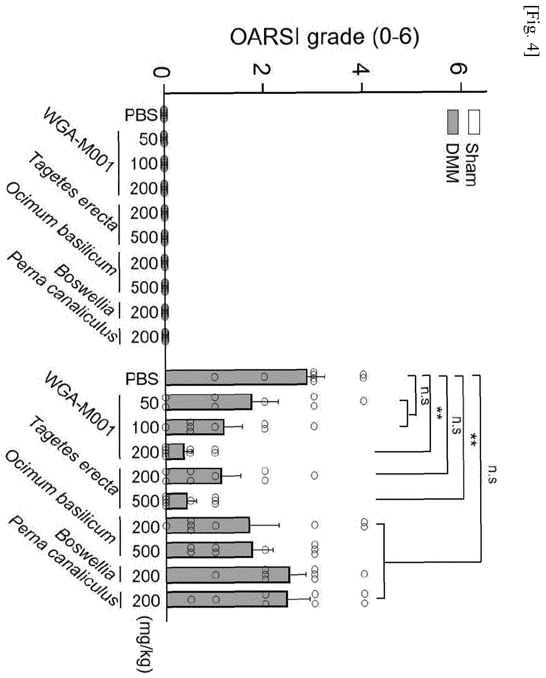

FIG. 4 is a graph showing the grades of osteoarthritis according to the concentration-dependent administration of experimental materials in mice in which osteoarthritis is induced by DMM, which are evaluated by the OARSI system. [Sham, normal group mice; DMM, mice with osteoarthritis induced by DMM; PBS, a negative control administered PBS; WGA-M001, a group administered a complex of a marigold extract and a basil extract; *Tagetes erecta,* a group administered only marigold extract; *Ocimum basilicum,* a group administered only basil extract; *Boswellia,* a positive control administered *Boswellia* extract; *Perna canaliculus,* a positive control administered green lipped mussel extract]. The value was represented as a mean ± SD, and evaluated using the Kruskal-Wallis test and the Mann-Whitney U test. Significant differences from the negative control (PBS) were represented as *P<0.05, **P<0.01, ***P<0.001, ****P<0.0001, and n.s=not significant.

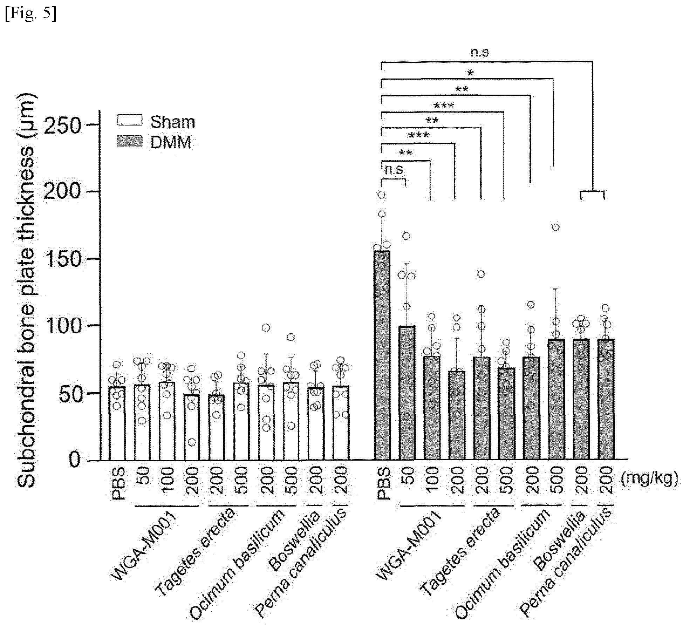

FIG. 5 is a graph showing subchondral bone plate (SBP) thicknesses according to concentration-dependent administration of experimental materials in mice with osteoarthritis induced by DMM. [Sham, normal group mice; DMM, mice with osteoarthritis induced by DMM; PBS, a negative control administered PBS; WGA-M001, a group administered a complex of a marigold extract and a basil extract; *Tagetes erecta,* a group administered only marigold extract; *Ocimum basilicum,* a group administered only basil extract; *Boswellia,* a positive control administered *Boswellia* extract; *Perna canaliculus,* a positive control administered green lipped mussel extract]. The value was represented as mean ± SD, and evaluated using the Kruskal-Wallis test and the Mann-Whitney U test. Significant differences from the negative control (PBS) were represented as *P<0.05, **P<0.01, ***P<0.001, ****P<0.0001, and n.s= not significant.

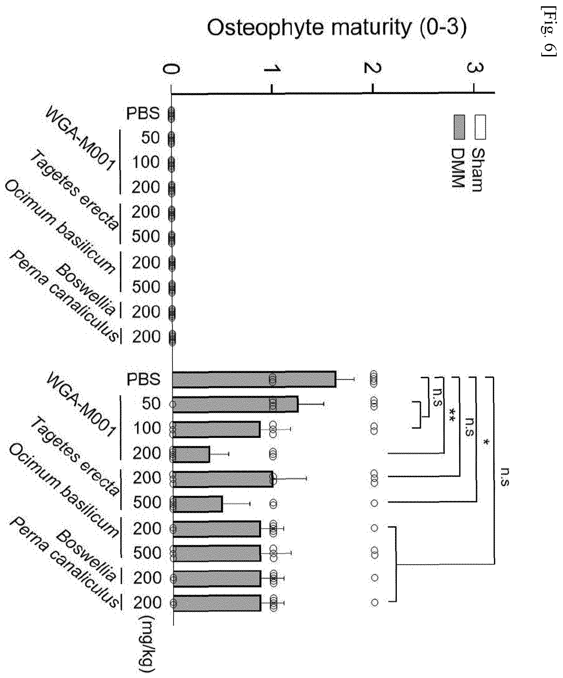

FIG. 6 is a graph evaluating the maturity (the degree of formation) of osteophytes according to concentration-dependent administration of experimental materials in mice with osteoarthritis induced by DMM. [Sham, normal group mice; DMM, mice with osteoarthritis induced by DMM; PBS, a negative control administered PBS; WGA-M001, a group administered a complex of a marigold extract and a basil extract; *Tagetes erecta,* a group administered only marigold extract; *Ocimum basilicum,* a group administered only basil extract; *Boswellia,* a positive control administered *Boswellia* extract; *Perna canaliculus,* a positive control administered green lipped mussel extract]. The value was represented as mean ± SD, and evaluated using the Kruskal-Wallis test and the Mann-Whitney U test. Significant differences from the negative control (PBS) were represented as *P<0.05, **P<0.01, ***P<0.001, ****P<0.0001, and n.s= not significant.

### [Modes of the Invention]

A composition of the present invention is characterized by containing marigold and basil extracts as active ingredients.

Marigold is an annual plant belonging to the genus *Tagetes* in the Asteraceae family, and is mainly used as a tea or as a flavoring or coloring agent for food. In the present invention, for example, one species of marigold selected from African marigold (*Tagetes erecta*)*,* French marigold (*T. patula*)*,* wild marigold (*T. minuta),* and Mexican marigold (*T. lucida),* preferably African marigold, may be used. In addition, in the present invention, the aerial part of the marigold may be used as an extractive raw material.

Basil is an annual plant belonging to the Lamiaceae family and is mainly used in Italian and French cuisine. In the present invention, for example, one species of basil selected from sweet basil (*Ocimum basilicum*), holy basil (*O*. *tenuiflorum*), American basil (*O*. *americanum),* Amazonian basil (*O*. *campechianum*), and African basil (*O*. *gratissimum*)*,* preferably sweet basil, is used. In addition, in the present invention, the aerial part of the basil may be used as an extractive raw material.

The term "extract" used herein may refer to an extraction product, including a liquid component obtained by immersing an extractive raw material in a solvent and extracting for a certain period of time at room temperature or in a heated state, or a solid content obtained by removing the solvent from the liquid component. In addition to the resulting products, the extract can be comprehensively interpreted to include dilutions and concentrated solutions thereof, and materials obtained by purifying the above products.

The extract of the present invention may be prepared according to an extraction method generally used in the art using marigold and basil as extractive raw materials. For example, extraction may be performed by using a polar solvent or a non-polar solvent. The polar solvent may be selected from the group consisting of C1 to C4 lower alcohols and water, and the non-polar solvent may be selected from the group consisting of chloroform, petroleum ether, ether, hexane, toluene, ethyl acetate, and dichloromethane. For the effects aimed at in the present invention, the extract of the present invention is preferably obtained using water, ethanol or a mixed solution thereof as a solvent, and more preferably using a mixed solution of water and ethanol as a solvent. The mixed solution of water and ethanol is preferably a 10 to 90%(v/v) ethanol aqueous solution, more preferably a 30 to 70%(v/v) ethanol aqueous solution, still more preferably a 40 to 60%(v/v) ethanol aqueous solution, and most preferably a 45 to 55%(v/v) ethanol aqueous solution.

During extraction, the solvent may be used at a weight, for example, 1 to 40 times, preferably 5 to 30 times, more preferably 10 to 30 times, still more preferably 10 to 25 times, and most preferably 15 to 20 times the dry weight of the extractive raw material.

An extraction temperature may be set to, for example, 0 to 120 °C, preferably 50 to 100 °C, more preferably 60 to 90 °C, still more preferably 70 to 90 °C, and most preferably 75 to 85 °C.

An extraction time may be set to, for example, 30 minutes to 12 hours, preferably 1 to 10 hours, more preferably 1 to 7 hours, still more preferably 1 to 4 hours, and most preferably 1 to 3 hours.

According to the above extraction conditions, an extract exhibiting the desired effect in the present invention can be efficiently prepared from marigold and basil.

In addition, the extract may be obtained by repeating extraction, for example, by repeating a process of isolating and recovering an extract obtained after first extraction from the residue, adding a new solvent to the residue, and obtaining an extract again through second extraction. When extraction is repeated, the number of repetitions may be, for example, 2 to 5 times, preferably 2 to 4 times, more preferably 2 to 3 times, and still more preferably 2 times.

To prepare an extract in a dried form, a drying process may be performed after extraction. For example, a freeze-drying or spray-drying method after concentration under reduced pressure is used. The concentration under reduced pressure is preferably performed at 50 to 70 °C under a pressure condition of 50 to 100 torr.

The marigold and basil extracts that are active ingredients of the present invention may be a mixture of a marigold extract and a basil extract, or may be extracted from a mixture of marigold and basil.

When the marigold extract and the basil extract are mixed, they may be mixed in a weight ratio ranging from, for example, 20:1 to 1:5, preferably 15:1 to 1:3, more preferably 12:1 to 1:2.5, and still more preferably 10:1 to 1:2.

When the marigold and basil extracts are extracted from the marigold-and-basil mixture, they may be extracts from the mixture in which marigold and basil are mixed in a weight ratio ranging from 20:1 to 1:5, preferably 15:1 to 1:3, more preferably 12:1 to 1:2.5, and still more preferably 10:1 to 1:2.

The composition of the present invention relates to a pharmaceutical composition for preventing or treating an inflammatory disease, and a functional food composition for preventing or improving an inflammatory disease, and the composition of the present invention may prevent, treat, or improve an inflammatory disease by the influence on inflammation-related factors, for example, by the action of inhibiting the increased expression of IL-6 and COX-2 due to the induction of an inflammatory response.

In the present invention, an inflammatory disease is a disease with inflammation as its main indication, and examples include an inflammatory lung disease, an inflammatory liver disease, an inflammatory bowel disease, an autoinflammatory disease, an inflammatory central nervous system disease, an inflammatory skin disease, and an allergic inflammatory disease as well as arthritis, but the present invention is not limited thereto.

The composition of the present invention is preferably a pharmaceutical composition for preventing or treating arthritis or a functional food composition for preventing or improving arthritis.

More preferably, the composition of the present invention is a pharmaceutical composition for preventing or treating degenerative arthritis or a functional food composition for preventing or improving degenerative arthritis. Particularly, the composition of the present invention may effectively prevent, treat or improve degenerative arthritis by the action of inhibiting the increased expression of MMP3 and MMP13 as well as the action of inhibiting the increased expression of IL-6 and COX-2.

The composition of the present invention is capable of being administered orally or parenterally during clinical administration and can be used in the form of a general pharmaceutical preparation. The pharmaceutical composition of the present invention may contain 0.1 to 100 wt% of the marigold and basil extracts as active ingredients with respect to the total weight of the composition, but the present invention is not limited thereto.

The pharmaceutical composition of the present invention may be formulated into a conventional pharmaceutical dosage form by selecting one or two or more pharmaceutically acceptable carriers and one or two or more additives in addition to the marigold and basil extracts that are active ingredients. The pharmaceutical composition of the present invention may be used alone or in combination with not only another pharmaceutically active compound but also an appropriate material.

The pharmaceutical composition of the present invention may be formulated into various oral or parenteral dosage forms. For preparation, the pharmaceutical composition of the present invention may be formulated using diluents or excipients such as commonly used fillers, thickening agents, binders, wetting agents, disintegrants, and surfactants. Solid preparations for oral administration include tablets, pills, powders, granules, and capsules, and such solid preparations are prepared by mixing at least one excipient, such as starch, calcium carbonate, sucrose, lactose, or gelatin, with the composition. Aside from simple excipients, lubricants such as magnesium stearate, talc, etc., are further used. Liquid preparations for oral administration include suspensions, liquids for internal use, emulsions, and syrups, and may further include various types of excipients, for example, a wetting agent, a sweetener, a fragrance and a preservative, other than commonly used simple diluents such as water or liquid paraffin. Preparations for parenteral administration include sterilized aqueous solutions, non-aqueous solvents, suspensions, emulsions, lyophilized formulations, and suppositories. As a non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin butter, or glycerogelatin may be used.

The pharmaceutical composition of the present invention may include compounds which contain vitamins B, C, E, or beta-carotene, minerals such as Ca, Mg, and Zn, phospholipids such as lecithin, or compounds such as maltol and amino acids, in addition to the active ingredients.

In addition, in addition to the above components, known additives may be used to enhance palatability, including natural flavors such as Japanese apricot, lemon, pineapple, or herbal flavors; natural juices; natural colorants such as chlorophyllin and flavonoids; sweetening agents such as fructose, honey, sugar alcohols, and sugar; and acidulants such as citric acid and sodium citrate.

Subjects to which the pharmaceutical composition of the present invention can be applied may be vertebrates, preferably mammals, and more preferably humans.

The pharmaceutical composition of the present invention may be administered orally or parenterally. The pharmaceutical composition of the present invention may be administered via parenteral routes, including topical application to the skin or injection into the peritoneal cavity, rectum, vein, muscle, subcutaneous tissue, uterine cavity, epidural space, or cerebral blood vessel, but is preferably administered orally.

The dosage of the pharmaceutical composition of the present invention varies depending on a patient's weight, age, sex, health condition, diet, time of administration, route of administration, excretion rate, and severity of a disease. For example, the daily dosage, based on the amount of marigold and basil extracts, may range from 50 to 5,000 mg/day for a 60 kg adult, and may be administered once daily or divided into 2 to 6 doses per day. According to the animal experimental results, the daily dosage may be 250 to 2,000 mg/day, preferably 500 to 2,000 mg/day, and more preferably 1,000 to 2,000 mg/day for a 60 kg adult based on the amount of the marigold and basil extracts.

The pharmaceutical composition of the present invention may further contain one or more active ingredients which exhibit the same or a similar function.

The pharmaceutical composition of the present invention may be used alone or in combination with surgery, radiation therapy, hormone therapy, chemotherapy, or methods using biological response modifiers.

A food composition of the present invention may be, for example, various food products such as chewing gum, caramel products, candies, frozen desserts, and snacks; beverage products such as soft drinks, mineral water, and alcoholic beverages; and health functional foods containing vitamins, minerals, etc.

The marigold and basil extracts of the present invention may be used alone or in combination with other food or food ingredients, and may be used appropriately according to a conventional method. The mixing amount of the active ingredients may be determined appropriately depending on the purpose of use. Generally, in the production of food or beverages, the active ingredients may be added at 15 wt% or less, and preferably 10 wt% or less with respect to the raw material. However, in case of long-term intake, the above amount may be the same as or lower than the above range, and the active ingredients may also be used in an amount more than the above range.

The food composition of the present invention may contain various flavorings or natural carbohydrates as additional ingredients. Natural carbohydrates may include monosaccharides such as glucose and fructose; disaccharides such as maltose and sucrose; polysaccharides such as dextrin and cyclodextrin; and sugar alcohols such as xylitol, sorbitol, erythritol, etc. As sweetening agents, natural sweetening agents such as thaumatin and stevia extracts, or synthetic sweetening agents such as saccharin and aspartame may be used.

Other than the above ingredients, the food composition of the present invention may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohols, and carbonating agents used in carbonated beverages. In addition, the food composition of the present invention may contain fruit pulp for the production of natural fruit juices, fruit juice beverages, and vegetable drinks. These ingredients may be used independently or in combination.

Hereinafter, the present invention will be described in further detail with reference to examples. However, these examples are merely provided to exemplify the present invention, and thus the scope of the present invention is not to be construed as being limited by these examples.

### Preparation example

### 1. Preparation of marigold extract

The aerial parts of marigold were washed with water to remove surface impurities, and cut and dried for subsequent use as an extraction material.

The prepared aerial parts of marigold and a 50%(v/v) ethanol aqueous solution in a volume 20 times larger than the aerial parts of the marigold were put into a reflux extraction device, and reflux-extracted twice at 80 °C for 2 hours.

The extract produced by reflux extraction was filtered, concentrated, powdered, and used as an experimental sample.

### 2. Preparation of basil extract

The aerial parts of basil were washed with water to remove surface impurities, and cut and dried for subsequent use as an extraction material.

The aerial parts of basil and a 50%(v/v) ethanol aqueous solution in a volume 20 times larger than the aerial parts of basil were put into a reflux extraction device, and reflux-extracted twice at 80 °C for 2 hours. Subsequently, the extract was filtered, concentrated, powdered, and used as an experimental sample.

### Example 1

The marigold and basil extracts prepared in the preparation example were mixed in a 1:1 weight ratio, and used as an experimental sample.

### Examples 2 to 5

The marigold and basil extracts prepared in the preparation example were mixed in a 4:1 weight ratio (Example 2), a 2:1 weight ratio (Example 3), a 1:0.1 weight ratio (10:1 weight ratio; Example 4), and a 1:2 weight ratio (Example 5), and used as experimental samples.

### Comparative Example 1

The marigold extract prepared in the preparation example was used alone as an experimental sample.

### Comparative Example 2

The basil extract prepared in the preparation example was used alone as an experimental sample.

### Experimental Example 1

The aim was to investigate the effect of each sample on the expression of key inflammation-related factors, such as COX-2 and IL-6, and catabolic factors causing degenerative arthritis, such as MMP3 and MMP13.

Chondrocytes isolated from mouse knee joints (primary chondrocytes) were used. ICR mouse P3 was used to isolate the articular chondrocytes from a mouse joint with collagenase, and the cells were seeded at 2.88 x 10⁵ per 35 mm dish and cultured in fetal bovine serum (FBS)/Dulbecco's modified Eagle medium (DMEM). When the cell confluence reached approximately 70%, IL-1β (1 ng/mL) and each sample were treated at various concentrations and incubated for 24 hours.

Then, qRT-PCR was performed to examine whether the expression of MMP3, MMP13, IL-6, and COX-2, which increased in the chondrocytes when inflammation (or degenerative arthritis) was induced by IL-1β treatment, was reduced by the sample treatment.

Each of the prepared samples was dissolved in DMSO and used for the experiment.

The experimental results are shown in FIGS. 1 and 2.

As shown in FIG. 1, the composition of Example 1, that is, the mixture of the marigold extract and the basil extract in a 1:1 weight ratio (in FIG. 1, represented as "marigold + basil"), inhibited the increased expression of MMP3, MMP13, IL-6 and COX-2 caused by inflammation (or degenerative arthritis). Particularly, as compared with Comparative Example 1 (in FIG. 1, represented as "marigold") and Comparative Example 2 (in FIG. 1, represented as "basil"), the composition of Example 1 exhibited a superior inhibitory effect on the increased expression of all four factors, demonstrating the synergistic effect of the combination of marigold and basil.

In addition, as shown in FIG. 2, all of the compositions of Examples 2 to 5 effectively inhibited the increased expression of MMP3, MMP13, IL-6 and COX-2, caused by inflammation (or degenerative arthritis) induced by IL-1β treatment, and exhibited the inhibitory effect on increased expression of all four factors at a level similar to that observed in Example 1.

### Experimental Example 2

To investigate whether each sample is effective in improving osteoarthritis in osteoarthritis animal models, a mouse, the most representative animal model of osteoarthritis, was selected and subjected to destabilization of medial meniscus (DMM) surgery to induce osteoarthritis.

For the DMM mouse model, DMM surgery, which involves removal of the fat pad and transection of the medial meniscotibial ligament, was performed in 10-week-old male C57BL/6 mice to increase mechanical stress on the knee joint and induce cartilage degradation and changes like osteoarthritis. After DMM surgery, the mice were randomly grouped into phosphate-buffered saline (PBS), Comparative Example 1 (marigold alone; represented as *"Tagetes erecta"*) (200 and 500 mg/kg), Comparative Example 2 (basil alone; represented as *"Ocimum basilicum"*) (200 and 500 mg/kg), and Example 4 (combination of marigold and basil; represented as "WGA-M001") (50, 100, and 200 mg/kg), and each test material was orally administered daily at the predetermined dose for 10 weeks to the groups of 8 mice (n=8 per group). Separately, to compare the efficacies of the test materials, as positive controls, health functional food ingredients that are currently widely used as health functional raw materials for joint health, such as *Boswellia* extract (represented as *"Boswellia"*) and green lipped mussel extract (represented as *"Perna canaliculus"*)*,* were orally administered daily at a dose of 200 mg/kg in the same manner as the test materials.

After 10 weeks had passed since the DMM surgery, a DMM-induced osteoarthritis mouse cartilage sample was taken and immobilized in 4% paraformaldehyde overnight, and then decalcified in 0.5M EDTA for 2 weeks. Subsequently, the sample was embedded in paraffin and the paraffin block was cut into a 5 µm thick section. The section was stained with hematoxylin, Safranin O and Fast Green. Histopathological outcomes were evaluated using the Osteoarthritis Research Society International (OARSI) grading system, osteophyte maturity grading, and measurement of subchondral bone plate (SBP) thickness. The representative safranin O-stained image was chosen based on a region showing the most advance lesion. OARSI scores were assessed in a blinded manner for the experimental groups. The OARSI grading system consists of seven grades (0 to 6), in which grade 0 indicates a joint with no sign of osteoarthritis, and grades 1 to 6 indicate various degrees of cartilage matrix abnormalities and the presence of the cells in the superficial zone. Specifically, these grades reflect surface discontinuity, vertical fissures, erosion, delamination, and deformation, and the details are shown in Table 1 below.

**[Table 1]**

| Criteria | Secondary grade | Grade |
|---|---|---|
| Intact surface and cartilage, no damage | No secondary grade | 0 |
| Matrix: intact surface layer, edema and/or | Intact surface | 1 |
| myofibroblasts: proliferation, hypertrophy | 1.0: Intact cells, 1.5: Apoptosis | |
| Reduced staining of the upper 1/3 of cartilage (middle zone) (Safranin O or Toluidine Blue), irregular cartilage columns | Discontinuous surface (rough) | 2 |
| | 2.0: Surface fibrillation, 2.5: Surface wear accompanied by matrix loss in superficial zone | |
| Reduced staining of the upper 2/3 of cartilage (deep zone) (Safranin O or Toluidine Blue), irregular cartilage columns | Vertical cracks/fissures | 3 |
| | 3.0: Simple cracks/fissures, 3.5: Irregular/complex fissures | |
| Loss of cartilage matrix, cyst formation within cartilage matrix | Erosion | 4 |
| | 4.0: Separation of superficial zone, 4.5: Collapse of middle zone | |
| Articular surface with repaired tissue including sclerotic bone or fibrous cartilage | Denudation | 5 |
| | 5.0: Intact bone surface, 5.5: Presence of repaired or recovered tissue | |
| Deformation of articular surface contour, including bone remodeling, microfractures, and repair | 6: Deformation 6.0: Presence of marginal osteophytes | 6 |
| | 6.5: presence of marginal and central osteophytes | |

As a result of the daily oral administration of the marigold-basil complex to the DMM-induced osteoarthritis mice to confirm its *in vivo* function, as seen from the Safranin O staining results in FIG 3, the marigold-basil complex (WGA-M001) exhibited a protective effect against osteoarthritis development. Particularly, both the marigold only (*Tagetes erecta*) and the basil only (*Ocimum basilicum*) must to be administered at high doses (500 mg/kg) to prevent osteoarthritis development, but the marigold-basil complex (WGA-M001) showed similar osteoarthritis inhibition and protective effects to the high-dose group in the 100 mg/kg administered group. In addition, in fact, the results of OARSI grading, SBP thickness, and bone tissue maturity measurements were consistent with those of the articular cartilage tissue analysis observed via Safranin O staining (FIGS. 4 to 6). This shows that the joint protective effect of the marigold-basil complex is at least 5-fold stronger than those of other extracts, and that the combined use of marigold and basil has a synergistic effect, demonstrating cartilage recovery effects even at lower concentrations compared to their individual use.

Referring to the PBS (negative control) in FIG. 3, the infiltration of inflammatory cells into joints and cartilage significantly increased in the mice of DMM-induced osteoarthritis as compared to the normal group (Sham). On the other hand, in the marigold extract (*Tagetes* erecta)-treated group, the basil extract (*Ocimum basilicum*)*-*treated group, and the marigold-basil complex (WGA-M001)-treated group, the inflammation response was alleviated, and particularly, in the marigold-basil complex (WGA-M001)-treated group, significant alleviation of the inflammation response could be confirmed.

In addition, as subchondral bone plate (SBP) thicknesses in the mice of osteoarthritis induced by DMM surgery were measured and found to have greatly increased in the negative control (PBS) not administered any experimental material, but were found to have greatly decreased in the marigold-basil complex (WGA-M001)-administered group. This result can show that the marigold-basil complex has an effect of improving osteoarthritis. In addition, the marigold-basil complex (WGA-M001) exhibited a greater inhibitory effect on osteoarthritis than the *Boswellia* extract (*Boswellia*) and the green lipped mussel extract (*Perna canaliculus*), which are currently used as health functional raw materials for joint health.

## Claims

1. A pharmaceutical composition for preventing or treating an inflammatory disease, containing marigold and basil extracts as active ingredients.

2. The pharmaceutical composition of claim 1, wherein the extract is a 10 to 90%(v/v) ethanol extract.

3. The pharmaceutical composition of claim 1, wherein the inflammatory disease is arthritis.

4. The pharmaceutical composition of claim 3, wherein the arthritis is degenerative arthritis.

5. The pharmaceutical composition of claim 1, wherein the extract is an extract of a mixture in which marigold and basil are mixed in a weight ratio ranging from 15:1 to 1:3.

6. A functional food composition for preventing or improving an inflammatory disease, containing marigold and basil extracts as active ingredients.

7. The functional food composition of claim 6, wherein the extract is a 10 to 90%(v/v) ethanol extract.

8. The functional food composition of claim 6, wherein the inflammatory disease is arthritis.

9. The functional food composition of claim 8, wherein the arthritis is degenerative arthritis.

10. The functional food composition of claim 6, wherein the extract is an extract of a mixture in which marigold and basil are mixed in a weight ratio ranging from 15:1 to 1:3.
